# EUROPEAN PATENT SPECIFICATION

(11) **EP 4 358 928 B1**
(45) Date of publication and mention of the grant of the patent: **22.10.2025**
(21) Application number: 22730147.0
(22) Date of filing: 18.05.2022
(51) Int. Cl.: A61K 8/34, A61K 8/37, A61K 8/46, A61K 8/49, A61K 8/67, A61K 8/73, A61K 8/81, A61Q 19/08

(54) **COSMETIC COMPOSITION WITH ENHANCED COLOR STABILITY**
KOSMETISCHE ZUSAMMENSETZUNG MIT ERHÖHTER FARBSTABILITÄT
COMPOSITION COSMÉTIQUE PRÉSENTANT UNE MEILLEURE STABILITÉ DE COULEUR

(30) Priority: 24.06.2021 EP 21181573
(43) Date of publication of application: 01.05.2024
(73) Proprietor: Unilever IP Holdings B.V., 6708 WH Wageningen (NL); Unilever Global IP Limited, Wirral, Merseyside CH62 4ZD (GB)
(72) Inventor: BEKTO, Hasiba, 6708 WH Wageningen (NL); HUANG, Lei, 6708 WH Wageningen (NL)
(74) Representative: James, Helen Sarah
(86) International application number: PCT/EP2022/063453
(87) International publication number: WO 2022/268415

(56) References cited:
- WO-A1-2017/194486
- WO-A1-2021/078611
- WO-A1-2021/228502
- WO-A1-2021/228503
- DATABASE GNPD [online] MINTEL; 24 April 2013 (2013-04-24), ANONYMOUS: "Lytera Skin Brightening Complex", XP055671463, retrieved from https://www.gnpd.com/sinatra/recordpage/2050726/ Database accession no. 2050726
- DATABASE GNPD [online] MINTEL; 4 January 2021 (2021-01-04), ANONYMOUS: "Double Action Serum", XP055833511, retrieved from https://www.gnpd.com/sinatra/recordpage/8366789/ Database accession no. 8366789
- DATABASE GNPD [online] MINTEL; 4 January 2021 (2021-01-04), ANONYMOUS: "Double Action Serum", XP055833511, retrieved from https://www.gnpd.com/sinatra/recordpage/8366789/ Database accession no. 8366789
- DATABASE GNPD [online] MINTEL; 24 April 2013 (2013-04-24), ANONYMOUS: "Lytera Skin Brightening Complex", XP055671463, retrieved from https://www.gnpd.com/sinatra/recordpage/2050726/ Database accession no. 2050726

## Description

### Technical Field

Disclosed herein are personal care compositions. More particularly, disclosed herein is a cosmetic composition containing alkylresorcinol, a functionalized heteroaromatic compound, and retinoic acid precursor in combination with antioxidant, compatible oil, and emulsifying polymer to provide for improved color stability of the composition.

### Background

Modern definitions of ideal skin presently include youthful and resilient skin having uniform color distribution and smooth texture throughout. However, human skin is subject to deterioration through, for example, dermatological conditions, environmental abuse (wind, air conditioning, sun exposure, pollution, blue light), or chronoaging. Aging individuals increasingly develop facial fine lines, wrinkles, yellowing or sallowness, sagging, hyperpigmentation, age spots and the general signs of aging. Thus, the demand for "anti-aging" or "pre-aging" cosmetic products that address such visible signs has grown greatly.

Cosmetic compositions featuring retinoic acid precursors, such as retinol, have become quite prominent in recent years. Retinol, also known as vitamin A, and many of its ester derivatives and retinoid family members including, for instance, retinyl propionate, can be effective in reducing fine lines and wrinkles, smoothing skin, and improving uneven skin tone among other benefits when applied topically. Notwithstanding the benefits of retinoids, the inclusion of retinoic acid precursors in cosmetic compositions often results in compositions having difficulties with stability.

In addition, many people often search, separately or even simultaneously, for cosmetic products providing even skin tone benefits. The industry is currently marked by an ever-present need for cosmetic even tone agents that have enhanced efficaciousness and stability. Examples of such agents include hydroquinone, arbutin, kojic acid and functionalized heteroaromatic compounds. Without intending to be bound or limited by theory, it is believed that compositions containing a functionalized heteroaromatic compound, (e.g., niacinamide and/or nicotinate) are useful for promoting even skin and skin hydration while reducing skin dullness and the appearance of fine lines, among other benefits. Resorcinols, particularly alkylresorcinols such as hexylresorcinol and ethylresorcinol, provide further examples of agents providing even tone benefits that may be used in topical applications for the skin.

Cosmetic compositions containing even tone agents such as hexylresorcinol have become quite prominent in recent years. However, alkylresorcinol-containing compositions tend to become discolored in response to a variety of factors such as temperature, light, and oxidation. Obtaining cosmetic compositions comprising resorcinols that are color stable is challenging. The issue of color instability in cosmetic compositions is exacerbated when resorcinols are found in the same composition as niacinamide and/or retinoid compounds, resulting in more prominent degradation of color when compared to compositions with each skin benefit agent independently. Thus, the color instability of cosmetic compositions with alkylresorcinols, functionalized heteroaromatic compound and retinoic acid precursors poses even further a challenge to be overcome.

Therefore, the present inventors have recognized a need to develop a cosmetic composition with improved color stability that includes alkylresorcinol, a functionalized heteroaromatic compound and retinoic acid precursor. Thus, disclosed herein is a cosmetic composition having an alkylresorcinol in combination with (a) functionalized heteroaromatic compound, (b) a retinoic acid precursor or (c) a mixture thereof in addition to emulsifying polymer, antioxidant, and a compatible oil.

### Additional Information

Efforts have been disclosed for incorporating emulsifying polymer into cosmetic compositions with resorcinols, functionalized heteroaromatic compounds, and/or retinoids.

U.S. Patent Application Publication No. 2003/165546 discloses stable personal care compositions containing a retinoid and Carbomer.

U.S. Patent No. 8,440,172 describes soluble salts of phenylbenzimidazole sulfonic acids at pH's at or below 7.0.

PCT Publication No. WO 2019/011618 relates to nanoemulsions with color stabilized actives, optionally including retinoic acid precursor, resorcinol, and/or polymer.

PCT Publication No. WO 2017/194486 reports a method of stabilizing retinoic acid precursors and a skin benefit composition with stabilized retinoic acid precursors.

PCT Publication No. WO 2021/078611 discloses a cosmetic composition comprising an alkylresorcinol, a retinoid, a compatible oil where the oil has a Hansen total solubility parameter from 16 to 22 and an antioxidant.

None of the additional information above describes the particular combination of emulsifying polymer with resorcinol, functionalized heteroaromatic compound, retinoid, antioxidant and a compatible oil to achieve a color stable cosmetic composition to be used to address signs of aging of the skin as disclosed herein.

### Summary

The present inventors have found that the use of an emulsifying polymer in addition to use of a compatible oil carrier and antioxidant enhance the color stability of cosmetic compositions comprising retinoic acid precursor, functionalized heteroaromatic compound and alkylresorcinol.

Accordingly, in a first aspect, the inventive composition relates to a composition according to claim 1.

The invention also relates to a method of providing an antiaging and/or even tone benefit to the skin comprising the steps of (a) making a color stable cosmetic composition comprising alkylresorcinol, functionalized heteroaromatic compound, and retinoic acid precursor according to claim 1 and (b) applying the cosmetic composition to skin in need of treatment is disclosed.

All other aspects of the present cosmetic compositions will become more readily apparent upon considering the detailed description and examples which follow.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above.

The term "skin" as used herein includes the skin on the face, neck, chest, back, arms, axilla, buttocks, hands, legs and scalp. Skin benefit agent, as used herein, is meant to include a component that improves a facial or body characteristic after topical application like a skin characteristic and/or benefits the same wherein the skin benefit agent can be, and preferably is, a skin benefit agent in a cream, pump or aerosol spray, serum, lotion, balm, deodorant, gel or wash composition. In an especially preferred embodiment, the composition is a leave-on composition and the skin benefit agents are retinoid compounds and oil-soluble alkylresorcinols. As used herein, "monomer" means a discrete, non-polymerized chemical moiety that undergoes polymerization in a chemical reaction to result in a macromolecule and "unit" refers to a monomer that has already undergone such a polymerization reaction and is part of a polymer. "Polymer" refers to the reaction product formed from the polymerization of at least two monomer units. The use of "emulsifying polymer" is to be used interchangeably with "polymeric emulsifier" herein and refers to a multifunctional polymeric ingredient that has the potential to emulsify, stabilize and/or build viscosity of a composition. The terms "retinoid" and "retinoic acid precursor," as used herein, refers to all natural and/or synthetic analogues of vitamin A or derivatives thereof having similar biological activity as vitamin A in human skin. The term "derivative(s)" as used with reference to ingredients herein refers to functional group substitutions on the compound. The term "functionalized," as used herein, means that a compound possesses at least one functional group substituent, including an acyl, hydroxy, alkoxy, carboxamide (amide), carboxyl, or carboalkoxy (ester) substituent, on the compound. Substantially free of, as used herein, is intended to mean comprising less than 5% by weight, preferably, less than 2% by weight, more preferably, less than 0.5% by weight of the composition. Remains color stable, as used herein, describes a cosmetic composition that possesses a ΔE of six (6) or less based on L*, a* and b* color differences taken on a Hunter Lab spectrophotometer at 4 weeks at 45°C. Generally, ΔE ≤6 at 4 weeks at 45°C is desirable and ΔE ≥6 is undesirable. The term "compatible" as used herein refers to an oil possessing a Hansen total solubility parameter value within the range of 16.5 to 22. Hansen total solubility parameters is determined based on three Hansen parameters, namely t (defines energy from dispersion forces between molecules), ₚ (defines energy from dipolar intermolecular forces between molecules), and ₙ (defines energy from hydrogen bonds between molecules). t values are obtained according to the CRC Handbook of Solubility Parameters and other Cohesion Parameters, Second Edition by Allan F.M. Barton. Ambient temperature, as used herein, refers to a temperature of 20 to 25°C.

Except in the examples, or where otherwise explicitly indicated, all numbers in this description indicating amounts of material are to be understood as modified by the word "about." All amounts are by total weight of the composition, unless otherwise specified.

### Detailed Description

Resorcinol is a dihydroxy phenol compound (i.e., 1,3-dihydroxybenzene) characterized by alcohol groups (-OH) at positions 1 and 3. The chemical structure of resorcinols may be modified, resulting in substituted resorcinols characterized by at least one substituent in the 2, 4, 5 or 6 position. It is preferred that the resorcinol comprises at least one substituent comprising 5 to 11 carbon atoms, preferably, 5 to 9 carbon atoms. It is particularly preferred that at least one substituent comprises an alkyl group, more preferably, an unsaturated alkyl group.

Preferably, the resorcinol of the cosmetic composition is only substituted at position 4 with an alkyl group, i.e., the resorcinol is an alkylresorcinol, more preferably, a 4-substituted alkylresorcinol. Illustrated but not limiting examples of 4-substituted alkylresorcinols include 4-ethylresorcinol, 4-hexylresorcinol, 4-phenylethylresorcinol, 4-cyclopentyl resorcinol, 4-cyclohexylresorcinol, 4-octylresorcinol, and mixtures thereof. The composition comprises 0.001 to 10%, preferably, 0.01 to 5%, and, most preferably, 0.01 to 1% alkylresorcinol by weight of the cosmetic composition, including all ranges subsumed therein. It is particularly preferred that the alkylresorcinol is 4-hexylresorcinol, 4-ethylresorcinol, or mixtures thereof.

The inventive compositions contain a retinoic acid precursor, selected from retinyl esters, retinol, retinal, or mixtures thereof, preferably, retinal, retinyl ester, or mixtures thereof. Examples of retinyl esters desirable for use include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl taurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, and retinyl oleate. Preferably, the ester is selected from retinyl palmitate, retinyl acetate, retinyl propionate, and mixtures thereof. Retinyl linoleate and retinyl oleate are also preferred. In a particularly preferred embodiment, the retinoic acid precursor is retinyl proprionate, retinol, or mixtures thereof. Retinoic acid precursor is employed in an amount of 0.001 to 5%, preferably, in an amount of 0.01 to 1%, most preferably, in an amount of 0.01 to 0.5% by weight of the cosmetic composition, including all ranges subsumed therein.

Cosmetic compositions disclosed herein include, in addition to alkylresorcinol and retinoic acid precursor, a functionalized heteroaromatic compound. The functionalized heteroaromatic compound comprises a carboxylic acid functionalized heteroaromatic compound comprising nicotinic acid, niacinamide, nicotinate, picolinic acid, picolinamide or mixtures thereof, and, more preferably, niacinamide, picolinamide or mixtures thereof. The functionalized heteroaromatic compound is typically present in an amount of 0.1 to 5% by weight of the cosmetic composition, including all ranges subsumed therein.

Now it has surprisingly been determined that use of a compatible oil in compositions with alkylresorcinol, retinoic acid precursor and a functionalized heteroaromatic compound can contribute to improved color stability. Oils with a Hansen total solubility parameter ( ₜ) value within the range of 16.75 to 20, more preferably, 17 to 19.5, are most compatible and thus desirable for use in such cosmetic compositions. Oils that are used in the compositions disclosed herein are isopropyl myristate ( t =17.5), caprylic/capric triglycerides ( t =18.9), isopropyl palmitate ( t =17.2) or mixtures thereof. Compatible oils are employed in an amount of 8 to 40% by weight, preferably, in an amount of 10 to 30%, and, most preferably, in an amount of 12 to 25% by weight of the cosmetic composition, including all ranges subsumed therein. In a particularly preferred aspect, the cosmetic composition comprises 15 to 20% by weight of compatible oil.

It is within the scope of the cosmetic compositions to further include one or more antioxidants, which may be natural, naturally derived, or synthetic antioxidants, as it has also unexpectedly been found to enhance color stability in such cosmetic compositions. Antioxidants may be any antioxidant desirable for use in cosmetic compositions. Illustrative but nonlimiting examples of such antioxidants comprise alpha hydroxy acids (e.g., citric acid, lactic acid, glycolic acid and mandelic acid), amino acids and derivatives thereof, beta-hydroxy acids (e.g., propanoic acid and salicylic acid), butylated hydroxytoluene (BHT), carotenes, carotenoids, co-enzyme Q10, didodecyl 3,3' thiodipropionate, glutathione, imidazole and derivatives thereof, octadecyl di-t-butyl-4-hydroxyhydrocinnamate, pentaerythrityl tetra-di-t-butyl hydroxyhydrocinnamate, peptides and derivatives thereof, plant extracts (e.g., *Rosmarinus officinalis* (rosemary) leaf extract and *Camellia sinesis* (green tea) leaf extract), polyhydroxy acids (e.g., gluconic acid, gluconolactone and lactobionic acid), polyphenols (e.g., anthocyanin, ellagic acid, flavonoid, tannin), and resveratrol.

Another class that may be used in the inventive cosmetic composition as antioxidants includes vitamins. Illustrative vitamins include, but are not limited to, vitamin B₂, vitamin B₅ (panthenol), vitamin B₆, vitamin C, vitamin E, vitamin F, vitamin K, folic acid and biotin. Derivatives of the vitamins may also be employed. For instance, vitamin C derivatives include ascorbyl tetraisopalmitate, magnesium ascorbyl phosphate and ascorbyl glycoside. Derivatives of vitamin E include tocopheryl acetate, tocopheryl palmitate and tocopheryl linoleate. It is preferred that the antioxidant comprises BHT, didodecyl 3,3' thiodipropionate, octadecyl di-t-butyl-4-hydroxyhydrocinnamate, pentaerythrityl tetra-di-t-butyl hydroxyhydrocinnamate, polyphenols, or a mixture thereof. The total weight percent of antioxidants present in the cosmetic compositions disclosed herein is preferably 0.01 to 4%, more preferably, 0.02 to 3%, and, most preferably, 0.05 to 2% by weight of the cosmetic composition, including all ranges subsumed therein. The minimum total weight percent of antioxidants present in the cosmetic compositions is 0.15%, preferably, 0.20% by weight based on the total weight of the composition.

It has further been found that when a retinoid, alkylresorcinol and functionalized heteroaromatic compound is added in combination with a compatible oil and antioxidant as above with an emulsifying polymer, still further increased color stability is unexpectedly observed when compared to comparable compositions lacking such emulsifying polymers. The emulsifying polymer comprises Acrylates/C₁₀₋₃₀ Alkyl Acrylate Crosspolymer, Carbomer, or a mixture thereof. The emulsifying polymer is multifunctional and is capable of emulsifying, stabilizing, and/or building viscosity of a composition. It is within the scope of the present compositions to employ a polymeric emulsifier alone or in combination with other additional polymeric emulsifiers. Illustrative but nonlimiting Acrylates/C₁₀₋₃₀ Alkyl Acrylate Crosspolymer for use in the present cosmetic compositions are made commercially available by the supplier The Lubrizol Corporation under such trade names as PEMULEN^{™} TR-1, PEMULEN^{™} TR-2, PEMULEN^{™} EZ-4U, CARBOPOL^{®} Ultrez 20, CARBOPOL^{®} Ultrez 21, CARBOPOL^{®} 1382 and CARBOPOL^{®} ETD 2020. Desirable Carbomers for use in the present compositions include CARBOPOL^{®} Ultrez 10 and CARBOPOL^{®} 980, both commercially sold by The Lubrizol Corporation. Emulsifying polymers are included in the cosmetic composition in an amount of 0.01 to 5%, preferably, from 0.02 to 4%, and, more preferably, from 0.03 to 3%, by total weight of the composition. It is still more preferred that the polymers are included in the cosmetic composition at 0.04 to 2% by weight, most preferably, 0.05 to 1% by weight of the cosmetic composition, including all ranges subsumed therein.

One of ordinary skill in the art would be adept to identify any need of neutralization for the emulsifying polymers disclosed herein to achieve a desirable and/or maximum viscosity. Any pH modifier known in the field acceptable for use in personal care and/or pharmaceutical products can be employed to result in a cosmetic composition possessing a skin-acceptable pH, typically within the range of 4 to 8. In a preferred embodiment, the pH of the cosmetic composition is 4.25 to 7.5, and most preferably, 5 to 7. Traditional buffers or pH modifiers include common additives such as sodium hydroxide, potassium hydroxide, hydrochloric acid, citric acid, triethanolamine and aminomethyl propanol. Viscosity of the cosmetic composition disclosed herein is preferably

1,000 to 120,000 centipoise (cps), and, more preferably, 5,000 to 80,000 cps, taken under conditions of ambient temperature and a shear rate of 1s⁻¹ with a strain controlled parallel plate rheometer made commercially available from suppliers like T.A. Instruments under the Discovery name. Alternatively, viscosity can also be measured using a Brookfield Viscometer (speed at 20 rpm, spindle 5, helipath off, for one (1) minute at ambient temperature). The cosmetic composition can be formulated as a serum having a viscosity of 1,000 to 4,000 millipascal-second (mPas), a lotion having a viscosity of 4,000 to 10,000 mPas, a fluid cream having a viscosity of 10,000 to 20,000 mPas or a cream having a viscosity of 20,000 to 100,000 mPas or above.

Cosmetic compositions disclosed herein are substantially free of additional surfactants, that is, cosmetic compositions comprise less than 5%, preferably, less than 2%, more preferably, less than 0.5% surfactant by weight of the composition, in part due to the emulsifying properties of the emulsifying polymer.

One of ordinary skill in the art, however, would recognize if there was a composition-specific need to include additional surfactants, which encompass the category of materials known in the relevant field as emulsifiers. Again, optional surfactants selected for use would not exceed a maximum inclusion level of 5%, preferably, not exceeding 2%, and, more preferably, not exceeding 0.5% surfactant by weight of the cosmetic composition. A skilled artisan would also be knowledgeable in the selection of appropriate surfactant or combination of surfactants required based on common factors including, but not limited to, hydrophilic-lipophilic balance (HLB) value of surfactants, pH of the cosmetic composition, and electrolyte content of the cosmetic composition.

The emulsifier may be selected from the group consisting of anionic, nonionic, cationic and amphoteric surfactants. Particularly preferred nonionic surfactants are those with a C₁₀-C₂₀ fatty alcohol or acid hydrophobe condensed with 2 to 100 moles of ethylene oxide or propylene oxide per mole of hydrophobe; C₂-C₁₀ alkyl phenols condensed with 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g., methyl gluconamides) are also suitable nonionic emulsifiers. Still other preferred nonionic surfactants include glyceryl stearate, glycol stearate and stearamide AMP.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ acyl methyl isethionates, C₈-C₂₀ acyl methyl taurates, C₈-C₂₀ alkyl ether phosphates, alkyl ether carboxylates and combinations thereof.

Cationic emulsifiers that may be used include, for example, palmitamidopropyltrimonium chloride, distearyldimonium chloride, diester quaternary ammonium compounds (e.g., distearoylethyl dimonium chloride), and mixtures thereof.

Color stability is determined through the use of LabScan XE equipment (Hunter Associates Laboratory, Inc. Reston, Virginia). LabScan XE measures color data. Samples at different storage stages housed in vials or in measurement cells are loaded onto the LabScan XE measurement port. By following the measurement procedure specified in the associated instrument menu and computer software, colors (a*, b* and L*) are measured and calculated to result in a color change data (ΔE) value. For reference, a ΔE of approximately three is the threshold where one may visibly detect a color change from the original sample.

It has also been unexpectedly discovered that other compositions made with the present cosmetic compositions provide good stability and color stability for the composition itself, as well as anti-aging and even tone benefits when applied to the skin.

Cosmetic compositions disclosed herein may also comprise a cosmetically acceptable vehicle to act as a diluent, dispersant or carrier for the skin benefit agents in the composition, so as to facilitate its distribution when the composition is applied to the skin. This cosmetically acceptable vehicle may be aqueous, anhydrous or an emulsion. Oily carriers in the presence of water and an emulsifier will form emulsion systems as carriers. Preferably, the compositions are aqueous or an emulsion, especially water-in-oil or oil-in-water emulsion, preferentially, oil-in-water emulsion. The cosmetic compositions ordinarily will be in but are not limited to cream or lotion form.

Suitable carriers employed include water, sea water, rosewater (e.g., *Rosa Damascena* flower Water), tea (e.g., *Camellia Sinensis* Leaf Water), *Aloe Barbadensis* (aloe vera) leaf juice, witch hazel (e.g., *Hamamelis Virginiana* extract), lavender water (e.g., *Lavendula Angustifolia* flower water), grape water (e.g., *Vitis Vinifera* fruit water and *Vitis Vinifera* juice). In the most preferred embodiment, water is the carrier. Amounts of water can be 5 to 85%, more preferably, 15 to 75%, and, most preferably, 30 to 70% by weight based on the weight of the cosmetic composition and including all ranges subsumed therein. The cosmetically acceptable vehicle can, in the absence of other cosmetic adjuncts, form the balance of the composition. Besides water, desirable carrier classes include, but are not limited to, silicones, polyhydric alcohols, fatty acids, hydrocarbons, triglycerides, waxes and thickening agents.

Silicones may be categorized into the volatile and nonvolatile variety. Amounts can be, for example, 0.01 to 8%, more preferably, 0.1 to 6% by weight of the composition. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Nonvolatile silicones useful in this composition include polyalkyl siloxanes, polyalkylaryl siloxanes and polyether siloxane copolymers. The essentially nonvolatile polyalkyl siloxanes useful herein include, for example, polydimethyl siloxanes with viscosities from 5 x 10⁻⁶ to 0.1 m²/s at 25°C. Emulsifying and non-emulsifying silicone elastomers are also suitable for use in the cosmetic compositions disclosed herein.

Conventional humectants, generally of the polyhydric alcohol-type materials, can be optionally included in the cosmetic compositions disclosed herein. Typical polyhydric alcohols include glycerol (i.e., glycerine or glycerin), propylene glycol, propanediol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol, and mixtures thereof. The most preferred embodiment includes glycerin, propylene glycol, or a mixture thereof. The amount of humectant employed can be 0.1 to 25%, preferably, 0.5 to 20%, and, more preferably, 1 to 15% by weight of the cosmetic composition.

Fatty acids may also be useful carriers. The term "fatty" refers to carbon chain lengths ranging from 10 to 30 carbon atoms. Illustrative of this category are pelargonic, lauric, myristic, palmitic, stearic, isostearic, hydroxystearic, oleic, linoleic, ricinoleic, arachidic, behenic, erucic acids and combinations thereof. When used, from 0.01 to 5% by weight fatty acid is present in the composition.

Waxes and wax esters are also suitable for use in the cosmetic compositions. These waxes include animal-derived waxes or those from animal byproducts (e.g., beeswax, spermaceti wax, Chinese wax, wool wax, shellac wax), plant-derived waxes (e.g., tribehenin wax, carnauba wax, candelilla wax, bayberry wax, jojoba wax, orange wax, rice bran wax, sunflower wax, castor wax, soy wax), mineral waxes (e.g., montan wax, ceresin wax, ozokerite) and synthetic derivatives of natural waxes. The amount of wax and wax esters employed can be 0.01 to 10%, preferably, 0.1 to 8% by weight of the composition.

Triglycerides are another group of materials useful as carriers, in addition to triglyceride oils having a Hansen total solubility parameter value within the scope of the inventive cosmetic composition that may be employed as compatible oils. Illustrative but not limiting examples are sunflower seed oil, cotton oil, canola oil, grapeseed oil, soybean oil, castor oil, borage oil, olive oil, shea butter, jojoba oil, and mixtures thereof. Mono- and di-glycerides may also be useful. Illustrative of these categories are glyceryl monostearate and glyceryl distearate. Amounts of triglycerides can be 0.01 to 10%, preferably, from 0.1 to 8% by weight of the composition.

The cosmetic compositions may optionally comprise one or more thickening agents in addition to the emulsifying polymer previously described herein, preferably, in an amount of 0.05 to 3%, more preferably, 0.1 to 2%, and, even more preferably, 0.2 to 1% by weight of the composition. Useful thickening agents include polysaccharides, which comprise of starches, natural/synthetic gums and cellulosics. Desirable starches include tapioca starch, cornstarch, potato starch, aluminum starch octenylsuccinate and sodium hydroxypropyl starch phosphate. Desirable gums include xanthan, sclerotium, pectin, karaya, arabic, agar, guar, carrageenan, alginate and combinations thereof. Desirable cellulosics include hydroxypropyl cellulose, hydroxypropyl methylcellulose, ethylcellulose and sodium carboxy methylcellulose. Synthetic polymers functioning as thickening agents are also desirable for use, including taurate copolymers.

Fragrances, skin benefit agents, fixatives and abrasives may optionally be included in cosmetic compositions disclosed herein. Skin benefit agents include but are not limited to opacifiers, colorants, humectants, emollients, occlusive agents, plant extracts, optical agents, even tone agents, anti-inflammatory agents, anti-acne agents, sunscreens, antioxidants, photostabilizers, surfactants, wrinkle-reducing agents, desquamation promoters, exfoliating agents, mixtures thereof or the like. Each of these substances may be present in an amount of 0.05 to 5%, preferably, 0.1 to 3% by weight of the cosmetic composition.

Hydrocarbons may be optionally included in the cosmetic composition. Desirable hydrocarbons may include mineral oil, petrolatum and polyalpha-olefins. Examples of preferred volatile hydrocarbons include polydecanes such as isodecane and isododecane and the C₇-C₈ through C₁₂-C₁₅ isoparaffins.

Retinoid boosters may also be useful ingredients to incorporate for use in the compositions disclosed herein, such as fatty acids and derivatives thereof. As described in several U.S. Patents to Granger et al. (U.S. Pat. Nos. 5,759,556, 5,756,109, 5,747,051, 5,716,627, 5,811,110, 5,536,740, 5,747,051, 5,599,548, 5,955092, 5,885,595, 5,759,556, 5,693,330, 7,959,913, 8,226,933 and 8,409,550), the term "retinoid boosters" is used to refer collectively to compounds that have been determined to enhance the enzymatic conversion of retinyl esters and retinol to retinoic acid as part of the natural retinol metabolic process in the skin's epidermis. The boosters alone or in combination with other booster compounds help to enhance efficacy of a retinoic acid precursor. The term "fatty" refers to carbon chain lengths ranging from 10 to 30 carbon atoms. Illustrative of this category are pelargonic, lauric, myristic, palmitic, stearic, isostearic, hydroxystearic, oleic, linoleic, ricinoleic, arachidic, behenic, erucic acids and combinations thereof. Preferred derivatives of such fatty acids include fatty alcohols, such as cetyl, lauryl, myristyl, palmitic, cetearyl, stearyl and oleyl alcohols; fatty esters, such as 2-ethyl-hexyl myristate, isopropyl stearate and isostearyl palmitate, triisopropyl trilinoleate and trilauryl citrate, lauryl palmitate, myristyl lactate, oleyl eurcate and stearyl oleate, coco-caprylate/caprate (a blend of coco-caprylate and coco-caprete), propylene glycol myristyl ether acetate, diisopropyl adipate and cetyl octanoate; and fatty amides, such as cocamide monoethanolamide (CMEA), castor oil monoethanolamide, linoleoyl monoethanolamide (LAMEA), palmitamide monoethanolamide and linoleamide diethanolamide.

Other illustrative but nonlimiting examples of such booster compounds include carotenoids, flavonoids, cyclic and non-cyclic fragrances, antimicotics (e.g., bifonazole, climbazole, clotrimazole, econazole, ketoconazole, miconazole), phospholipid analogues, ureas, phosphatiylethanolamine, phosphatidylcholine, sphingomyelin, natural colorants (e.g., coumarin), quinolines, isoquinolines, metyrapone, and mixtures thereof. In an embodiment, fatty acids, fatty alcohols, fatty amides, climbazole, or a mixture thereof may be used as retinoid boosters. In a particularly preferred embodiment, 12-hydroxystearic acid, cetyl alcohol, cetearyl alcohol, CMEA, LAMEA, climbazole, or a mixture thereof is used as retinoid boosters. When used, 0.01 to 5% by weight of a retinoid booster compound is present in the composition.

It is within the scope of the cosmetic compositions to optionally include sunscreens and photostabilizers. The sunscreens and photostabilizers that may be used in the cosmetic compositions include such materials as octylmethoxycinnamate (OMK), ethylhexyl salicylate, phenylbenzimidazole sulfonic acid (Ensulizole), ethylhexyl p-methoxycinnamate, available as PARSOL MCX^{®}, Avobenzene (butyl methoxydibenzoylmethane), available as PARSOL 1789^{®} and benzophenone-3, also known as oxybenzone. Inorganic sunscreen actives may be employed such as microfine titanium dioxide (preferably, with a particle diameter of less than 150 nanometers (nm), and, most preferably, less than 100 nm) and zinc oxide may be used, polyethylene and various other polymers are also desirable sunscreens. Other sunscreens desirable for use include p-aminobenzoic acid (PABA), octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, mixtures thereof or the like. Also desirable for use is octocrylene. Amounts of the sunscreen or photostabilizing agents, when present, may generally be present in an amount of 0.1 to 20%, preferably, 0.5 to 15%, optimally, 0.75 to 10% by weight of the cosmetic composition.

Other additional optional skin benefit agents desirable for use include minerals and skin nutrients such as milk; magnesium, calcium, copper, zinc and other metallic components; kojic acid; hydroquinone and arbutin; saccharide isomerate; undecylenoyl phenylalanine; sphingolipids, phospholipids (e.g., phytosphingosine), ceramides (e.g. Ceramide 1, Ceramide 3, Ceramide 3B and Ceramide 6) and pseudoceramides; allantoin; pyroglutamic acid (PCA) salt derivatives including zinc PCA and sodium PCA; petroselinic acid; conjugated linoleic acid; octadecanoic acid; hyaluronic acid and its salt derivatives; and mixtures thereof and the like. Such skin benefit agents, when used, collectively make up 0.001 to 12% by weight of the cosmetic composition.

A wide selection of botanical extracts may optionally be included in the cosmetic compositions. The extracts may either be soluble in water or oil, carried in a solvent that is hydrophilic or hydrophobic, respectively. In a preferred embodiment, water or ethanol are the extract solvents. Illustrative examples include those extracted from green tea, yarrow, chamomile, licorice, aloe vera, citrus unshui, willow bark, alfalfa, algae, witch hazel, sage, thyme and rosemary, as well as oils such as those derived from sea buckthorn, moringa, argan, avocado, calendula, algal and marula. Soy extracts may be used and especially when it is desirable to include retinol. Such extracts, when used, are preferably employed in collective amounts of 0.001 to 12% by weight of the cosmetic composition.

Another optional additive desirable for use includes hemp oil with 2.5 to 25% by weight cannabigerol and/or cannabidiol at from 0.5 to 10 percent by weight. When used, such oil makes up 0.0001 to 12%, and, preferably, 0.01 to 5% by weight of the cosmetic composition.

Optionally, one or more coloring agents may also be included in the cosmetic compositions disclosed herein. Coloring agents include dyes or pigments of natural or synthetic origin. A dye selected for use may be organic or inorganic and water-soluble or oil-soluble. Illustrative and nonlimiting examples of water-soluble dyes include, for example, D&C Yellow 8, D&C Yellow 10, D&C Orange 4, D&C Red 6, D&C Red 22, D&C Red 28, D&C Red 33, D&C Green 5 or methylene blue. Oil-soluble dyes include, for example, D&C Red 17, D&C Green 6, β-carotene, D&C Violet 2, D&C Yellow 11, D&C Orange 5 and quinoline yellow. Pigments desirable for use are specially chosen from the mineral pigments, organic pigments, lakes, and mixtures thereof as known in the art. Illustrative but nonlimiting examples include mineral pigments such as titanium dioxide, ochres (yellow ochre, red ochre, brown ochre, iron hydroxide), metal oxides (zinc oxide, zirconium oxide, iron oxide, cerium oxide, chromium oxide), manganese violet, ultramarine blue, chromium hydrate and ferric blue, or metal powders (aluminum, bronze or copper powder); organic pigments such as nitroso, nitro, azo, phthalocyanine, diazine, xanthene, pyrene, quinoline, anthraquinone, triphenylmethane, fluorane, quinacridone, metal complex, isoindolinone, isoindoline, perinone, perylene, diketopyrrolopyrrole, indigo, thioindigo, dioxazine, triphenylmethane and quinophthalone compounds; and lakes (particularly those deriving from calcium, barium, aluminum and strontium salts) such as D&C Red No. 6 Barium Lake, D&C Red No. 7 Calcium Lake, D&C Red No. 27 Aluminum Lake, D&C Red No. 28 Aluminum Lake, D&C Red No. 33 Aluminum Lake, FD&C Red No. 40 Aluminum Lake, FD&C Yellow No. 5 Aluminum Lake, FD&C Yellow No. 6 Aluminum Lake, D&C Yellow No. 10 Aluminum Lake, D&C Orange No.5 Aluminum Lake and F&D Blue No. 1 Aluminum Lake. Nacres, such as natural mica coated with a metal oxide, bismuth oxychloride or a natural pigment, are also desirable to be included in the cosmetic compositions disclosed herein. Other coloring agents desirable for use include chalk, activated charcoal and carbon black. Cosmetic compositions comprise 0.01 to 20%, preferably, 0.1 to 15% and, more preferably, 0.5 to 10% by weight of the coloring agent, when used, relative to the weight of the cosmetic composition.

Preservatives can be incorporated into the present cosmetic compositions as desired to protect against the growth of potentially harmful microorganisms. Cosmetic chemists are familiar with appropriate preservatives and routinely choose them to satisfy preservative tests and product stability tests. Preservative systems should be selected having regard for the use of the composition and possible incompatibilities between the preservatives and other ingredients in the formulation. Exemplary examples of preservatives for cosmetic compositions disclosed herein include, without limitations, iodopropynyl butyl carbamate (IPBC), phenoxyethanol, ethylhexylglycerine, 1,2-octanediol, methyl paraben, propyl paraben, imidazolidinyl urea, sodium dehydroacetate, propanediol, alkyl esters of para-hydroxybenzoic acid, hydroxyacetophenone, DMDM hydantoin derivatives, climbazole, propionate salts, and a variety of quaternary ammonium compounds. Preservatives are preferably employed in amounts of 0.01 to 2% by weight of the composition.

When making the present cosmetic composition, the desired ingredients are mixed via conventional methods. If applicable, the oil phase and water phases are separately mixed using standard mixers and mixing blades and heated to a temperature usually from 22 to 85°C under atmospheric pressure. Upon reaching the desired temperature, the oil phase is added to the water phase to create an emulsion. Other ingredients in the cosmetic composition are subsequently added and mixed in no particular order.

The cosmetic composition disclosed herein is a composition suitable for topical application to human skin, including leave-on and wash-off products. Preferably, the term encompasses a fluid liquid, and particularly a moisturizer rather than a make-up product. Most preferred are leave-on compositions. The term "leave-on" as used with reference to compositions herein means a composition that is applied to or rubbed on the skin and left thereon.

Many types of packaging can be used to store and deliver the present cosmetic composition. The selection of packaging is dependent upon the personal care end-use and the viscosity of the composition itself. As an example, leave-on lotions and creams for skin typically employ plastic containers with an opening at a dispense end covered by an appropriate closure. Conventional closures include flip-top hinged lids, screw-caps and non-aerosol pumps. As another example, appropriate packaging to be used for antiperspirants, deodorants and depilatories include a container with a roller-ball applicator on a dispensing end if the composition is fluid and of a thinner viscosity. If the composition is in a stick format, a container with a propel-repel mechanism wherein the stick is fixed on a platform towards a dispensing orifice is appropriate. If the composition is in an aerosol format, then metallic cans pressurized by a propellant and having a spray nozzle is appropriate. In general, patches, bottles, tubes, roller-ball applicators, squeeze containers or lidded jars are preferred.

In another aspect, the inventive composition relates to a method of providing an antiaging and/or even tone benefit to the skin comprising the steps of (a) making a color stable cosmetic composition comprising alkylresorcinol, functionalized heteroaromatic compound, and retinoic acid precursor according to the second aspect and (b) applying the cosmetic composition to skin in need of treatment.

The cosmetic compositions will now be described below in the context of specific non-limiting examples to facilitate a greater understanding of the present cosmetic compositions. One of ordinary skill in the art will recognize that variations of the inventive compositions that differ from the examples given may be practiced without deviating from the teachings of the present compositions.

### Examples

All samples were made by mixing the mentioned ingredients under conditions of moderate sheer, 22°C to 85°C, and atmospheric pressure. All □ₜ data were collected from the CRC Handbook of Solubility Parameters and Other Cohesion Parameters, Second Edition (by Allan F.M Barton).

### Example 1: Formulation with 3% niacinamide, 0.4% 4-hexylresorcinol and 0.3% retinyl propionate

| Ingredient | Weight percent (wt%) |
|---|---|
| Water | Balance |
| Glycerin | 3.50 |
| Chelator | 0.10 |
| Polymer/thickener | 0.50 |
| Niacinamide | 3.00 |
| Neutralizer | 0.20 |
| Preservative | 0.20 |
| Cetyl alcohol | 0.50 |
| Emulsifiers | 0.50 |
| Antioxidants | 0.20 |
| Oil | 15.0 |
| 4-Hexylresorcinol | 0.40 |
| Retinyl propionate | 0.30 |

An emulsion cosmetic composition was made by combining the above ingredients in accordance with the present cosmetic compositions. The inclusion levels and specific identities of antioxidants, oils and polymer/thickener selected may be varied to observe the impact of each factor on the color stability of the composition.

### Example 2: Different types of surfactant evaluated in composition

| | wt% | | | |
|---|---|---|---|---|
| | 1 | 2 | 3 | 4 |
| *Phase A- Water Phase* | | | | |
| Water | Balance | Balance | Balance | Balance |
| Chelator | 0.1 | 0.1 | 0.1 | 0.1 |
| Acrylates/C₁₀₋₃₀ Alkyl Acrylate Crosspolymer | 0.5 | 0.5 | 0.5 | 0.5 |
| 45% Potassium Hydroxide | 0.2 | 0.2 | 0.2 | 0.2 |
| Glycerin | 3.5 | 3.5 | 3.5 | 3.5 |
| **Polysorbate 20** | 0.5 | 0 | - | - |
| Niacinamide | 3 | 3 | 3 | 3 |

| *Phase B - Oil Phase* | | | | |
|---|---|---|---|---|
| Isopropyl Myristate | 7.5 | 7.5 | 7.5 | 7.5 |
| Cetyl Alcohol | 0.5 | 0.5 | 0.5 | 0.5 |
| **PEG-100 Stearate** | - | - | 0.5 | - |
| **Steareth-21** | - | - | - | 1.5 |
| **Steareth-2** | - | - | - | 1 |
| Pentaerythrityl tetra-di-t-butyl hydroxyhydrocinnamate | 0.1 | 0.1 | 0.1 | 0.1 |
| Didodecyl 3,3' thiodipropionate | 0.1 | 0.1 | 0.1 | 0.1 |

| *Phase C - post mixes* | | | | |
|---|---|---|---|---|
| Retinyl Propionate | 0.33 | 0.33 | 0.33 | 0.33 |
| Isopropyl Myristate | 2.5 | 2.5 | 2.5 | 2.5 |
| 4-Hexylresorcinol | 0.4 | 0.4 | 0.4 | 0.4 |
| Caprylic/capric triglyceride | 5 | 5 | 5 | 5 |
| Preservative | 0.2 | 0.2 | 0.2 | 0.2 |
| | | | | |
| ΔE after 4 weeks at 45°C | 2.9 | 5.7 | 4.2 | <6.5 |

Example 2 demonstrated that compositions comprising alkylresorcinol (e.g., 4-hexylresorcinol), functionalized heteroaromatic compound (e.g., niacinamide) and a retinoic acid precursor (e.g., retinyl propionate) in a compatible oil carrier with antioxidant and emulsifying polymer resulted in desirable ΔE values regardless of surfactant choice in such compositions after being subjected to 45°C conditions for 4 weeks. Sample 1 was the lead composition illustrative of the presently disclosed cosmetic compositions, exemplifying an excellent ΔE value of 2.9. Sample 1 is referenced in subsequent examples as a positive control against which other samples are compared and analyzed. In summary, the data in Example 2 showed that identity of surfactants found in the cosmetic composition did not greatly contribute to whether the composition possessed improved color stability.

### Example 3: Different types of emulsifying polymers evaluated in composition

| Sample | Chemical (INCI) Name | Trade Name/Supplier | wt% | ΔE (4 weeks @ 45°C) |
|---|---|---|---|---|
| 1 | Acrylates/C₁₀₋₃₀ Alkyl Acrylate Crosspolymer | PEMULEN^{™} EZ-4U/ Lubrizol | 0.5 | 2.45 |
| 1a | Acrylates/C₁₀₋₃₀ Alkyl Acrylate Crosspolymer | PEMULEN^{™} EZ-4U/ Lubrizol | 0.2 | 2.36 |
| 1b | Acrylates/C₁₀₋₃₀ Alkyl Acrylate Crosspolymer | PEMULEN^{™} EZ-4U/ Lubrizol | 0.75 | 2.50 |
| 5 | Acrylates/C₁₀₋₃₀ Alkyl Acrylate Crosspolymer | PEMULEN^{™} TR-1/ Lubrizol | 0.5 | 3.11 |
| 6 | Acrylates/C₁₀₋₃₀ Alkyl Acrylate Crosspolymer | PEMULEN^{™} TR-2/ Lubrizol | 0.5 | 3.14 |
| 7 | Acrylates/C₁₀₋₃₀ Alkyl Acrylate Crosspolymer | CARBOPOL^{®} Ultrez 20/ Lubrizol | 0.5 | 3.70 |
| 8 | Acrylates/C₁₀₋₃₀ Alkyl Acrylate Crosspolymer | CARBOPOL^{®} Ultrez 21/ Lubrizol | 0.5 | 3.73 |
| 9 | Acrylates/C₁₀₋₃₀ Alkyl Acrylate Crosspolymer | CARBOPOL^{®} 1382/ Lubrizol | 0.5 | 3.89 |
| 10 | Carbomer | CARBOPOL^{®} Ultrez 10/ Lubrizol | 0.5 | 3.23 |
| 11 | Carbomer | CARBOPOL^{®} 980/ Lubrizol | 0.5 | 3.61 |
| 12 | Ammonium Acryloyldimethyltaurate/Beheneth-25 Methacrylate Crosspolymer | ARISTOFLEX^{®} HMB/ Clariant | 0.75 | 8.11 |
| 13 | Ammonium Acryloyldimethyltaurate/VP Copolymer | ARISTOFLEX^{®} AVC/ Clariant | 0.75 | >6.5 |
| 14 | Polyacrylate Crosspolymer-6 | SEPIMAX^{™} Zen/ Seppic | 0.75 | 8.49 |
| 15 | Polyacrylamide & C₁₃-₁₄ Isoparaffin & Laureth-7 | SEPIGEL^{™} 305/ Seppic | 2-3 | N/A, separation |
| 16 | Polyphosphorylcholine Glycol Acrylate | PHOSPHOMER^{®} CEM510CNKC/ KCI Limited | 0.5 | 7.1 |
| 17 | Acrylates/Steareth-20 Methacrylate Copolymer | ACULYN^{™} 22/ Dow Inc. | 3.5 | 10.3 |
| 18 | Acrylates Copolymer | ACULYN^{™} 33/ Dow Inc. | 3 | N/A, separation |
| 19 | Sodium starch octenylsuccinate and Hydroxypropyl starch phosphate | STARDESIGN^{™} Power/ Cargill Beauty | 3 | N/A, separation |

All samples evaluated in Example 3 included 0.2 wt% antioxidant and 15 wt% compatible oils in combination with 0.4 wt% 4-hexylresorcinol, 3 wt% niacinamide and 0.33 wt% retinyl proprionate. Polymers were included in all samples at an amount sufficient to achieve a serum-like viscosity, so certain polymers (e.g., ACULYN^{™} 33) were used at higher concentrations to build desired viscosity and structure high loads of oily components. Samples 1 and 5 through 11, despite varying the type of emulsifying polymer, resulted in desirable ΔE values and such polymers were unexpectedly found to be of the Acrylates/C₁₀₋₃₀ Alkyl Acrylate Crosspolymer or Carbomer chemical name. Samples 1, 1a, and 1b were formulated by varying the inclusion levels of Acrylates/C₁₀₋₃₀ Alkyl Acrylate Crosspolymer, specifically PEMULEN^{™} EZ-4U, and all demonstrated excellent color stability (i.e., exhibited a ΔE value lower than 3) at 4 weeks at 45°C conditions. Samples 12 through 19, however, comprised emulsifying polymers outside the scope of the present cosmetic compositions and did not result in desirable ΔE values, if ΔE values were able to be measured at all, considering separation was observed for samples 15, 18 and 19.

### Example 4: Different types and amounts of antioxidants evaluated in composition

| | wt% | | | | |
|---|---|---|---|---|---|
| | 1 | 20 | 21 | 22 | 23 |
| *Phase A- Water Phase* | | | | | |
| Water | Balance | Balance | Balance | Balance | Balance |
| Chelator | 0.1 | 0.1 | 0.1 | 0.1 | 0.1 |
| Acrylates/C₁₀₋₃₀ Alkyl Acrylate Crosspolymer | 0.5 | 0.5 | 0.5 | 0.5 | 0.5 |
| 45% Potassium Hydroxide | 0.2 | 0.2 | 0.2 | 0.2 | 0.2 |
| Glycerin | 3.5 | 3.5 | 3.5 | 3.5 | 3.5 |
| Polysorbate 20 | 0.5 | 0.5 | 0.5 | 0.5 | 0.5 |
| Niacinamide | 3 | 3 | 3 | 3 | 3 |

| *Phase B - Oil Phase* | | | | | |
|---|---|---|---|---|---|
| Isopropyl Myristate | 7.5 | 7.5 | 7.5 | 7.5 | 7.5 |
| Cetyl Alcohol | 0.5 | 0.5 | 0.5 | 0.5 | 0.5 |
| **Pentaerythrityl tetra-di-t-butyl hydroxyhydrocinnamate** | 0.1 | 0 | 0.05 | - | - |
| **Didodecyl 3,3' thiodipropionate** | 0.1 | 0 | 0.05 | 0.1 | - |
| **Butylated hydroxytoluene (BHT)** | - | - | - | 0.1 | 0.2 |

| *Phase C - post mixes* | | | | | |
|---|---|---|---|---|---|
| Retinyl Propionate | 0.33 | 0.33 | 0.33 | 0.33 | 0.33 |
| Isopropyl Myristate | 2.5 | 2.5 | 2.5 | 2.5 | 2.5 |
| 4-Hexylresorcinol | 0.4 | 0.4 | 0.4 | 0.4 | 0.4 |
| Caprylic/capric triglyceride | 5 | 5 | 5 | 5 | 5 |
| Preservative | 0.2 | 0.2 | 0.2 | 0.2 | 0.2 |
| | | | | | |
| ΔE after 4 weeks at 45°C | 2.9 | 10.3 | 6.0 | 2.8 | 5.6 |

Experiments were conducted to evaluate the impact of varying the identity of and the inclusion level of antioxidants in the cosmetic compositions, where results are displayed in Example 4. All samples presented in Example 4 comprised constant types and concentrations of compatible oil in combination with 0.4 wt% 4-hexylresorcinol, 3 wt% niacinamide and 0.33 wt% retinyl propionate. In sample 20, which lacked any antioxidants, the ΔE value was measured at 10.3, beyond the acceptable ΔE limit of 6. In sample 21, when compared to positive control sample 1, the inclusion level of each antioxidant (i.e., pentaerythrityl tetra-di-t-butyl hydroxyhydrocinnamate and didodecyl 3,3' thiodipropionate) was reduced by half, but an acceptable ΔE value of 6 was still observed. In samples 22 and 23, desirable color stability of the compositions resulted despite varying combinations of antioxidants.

### Example 5: Different types and amounts of compatible oils evaluated in composition

| | wt% | | | | | | | |
|---|---|---|---|---|---|---|---|---|
| | 1 | 24 | 25 | 26 | 27 | 28 | 29 | 30 |
| *Phase A- Water Phase* | | | | | | | | |
| Water | Balance | Balance | Balance | Balance | Balance | Balance | Balance | Balance |
| Chelator | 0.1 | 0.1 | 0.1 | 0.1 | 0.1 | 0.1 | 0.1 | 0.1 |
| Acrylates/C₁₀₋₃₀ Alkyl Acrylate Crosspolymer | 0.5 | 0.5 | 0.5 | 0.5 | 0.5 | 0.5 | 0.5 | 0.5 |
| 45% Potassium Hydroxide | 0.2 | 0.2 | 0.2 | 0.2 | 0.2 | 0.2 | 0.2 | 0.2 |
| Glycerin | 3.5 | 3.5 | 3.5 | 3.5 | 3.5 | 3.5 | 3.5 | 3.5 |
| Polysorbate 20 | 0.5 | 0.5 | 0.5 | 0.5 | 0.5 | 0.5 | 0.5 | 0.5 |
| Niacinamide | 3 | 3 | 3 | 3 | 3 | 3 | 3 | 3 |

| *Phase B - Oil Phase* | | | | | | | | |
|---|---|---|---|---|---|---|---|---|
| **Isopropyl Myristate** (□ₜ =17.5) | 7.5 | 2.5 | 7.5 | - | - | - | 2 | 2.5 |
| **Isopropyl Palmitate** (□ₜ =17.2) | - | - | - | 15 | - | - | - | - |
| **Dimethicone 5 cSt** (□ₜ = 6.0) | - | - | - | - | 15 | - | - | - |
| **Isohexadecane** (□ₜ = 16.2) | - | - | - | - | - | 15 | - | - |
| Cetyl Alcohol | 0.5 | 0.5 | 0.5 | 0.5 | 0.5 | 0.5 | 0.5 | 0.5 |
| Pentaerythrityl tetra-di-t-butyl hydroxyhydrocinnamate | 0.1 | 0.1 | 0.1 | 0.1 | 0.1 | 0.1 | 0.1 | 0.1 |
| Didodecyl 3,3' thiodipropionate | 0.1 | 0.1 | 0.1 | 0.1 | 0.1 | 0.1 | 0.1 | 0.1 |

| *Phase C* - *post mixes* | | | | | | | | |
|---|---|---|---|---|---|---|---|---|
| Retinyl Propionate | 0.33 | 0.33 | 0.33 | 0.33 | 0.33 | 0.33 | 0.33 | 0.33 |
| **Isopropyl Myristate** (□ₜ =17.5) | 2.5 | 2.5 | 2.5 | 2.5 | 2.5 | - | 1.5 | 2.5 |
| 4-Hexylresorcinol | 0.4 | 0.4 | 0.4 | 0.4 | 0.4 | 0.4 | 0.4 | 0.4 |
| **Caprylic/capric triglyceride** (□ₜ =18.9) | 5 | 5 | 10 | - | - | - | 1.5 | 2.5 |
| Preservative | 0.2 | 0.2 | 0.2 | 0.2 | 0.2 | 0.2 | 0.2 | 0.2 |
| | | | | | | | | |
| ΔE after 4 weeks at 45°C | 2.9 | 5.8 | 2.3 | 3.3 | 24.5 | 16.4 | 10.4 | 7.3 |

The samples illustrated in Example 5 evaluated the impact of varying the type and inclusion levels of a compatible oil carrier suitable for use according to the present cosmetic compositions while holding constant the type and inclusion levels of antioxidant, emulsifying polymer, alkylresorcinol, functionalized heteroaromatic compound, and retinoic acid precursor.

Total amount of compatible oil for sample 24 was 10% by weight of the composition while that in sample 25 was 20% by weight of the composition. Both sample 24 and 25 resulted in ΔE values within the desired range. Therefore, Example 5 showed that use of compatible oils at 10 to 20% by weight of the composition in total resulted in a desirable ΔE value and thus color stability. Samples 26 to 28 varied the type of oil at consistent levels of 15%, wherein only sample 26 with isopropyl palmitate resulted in a ΔE value in the acceptable range, while samples 27 and 28 with dimethicone and isohexadecane, respectively, resulted in ΔE values outside of the acceptable range. Total amount of compatible oil for sample 29 was 5% by weight of the composition while that in sample 30 was 7.5% by weight of the composition. As both samples 29 and 30 were made outside the scope of the disclosed cosmetic compositions, the resulting ΔE values exceeded the desired upper limit of 6.

## Claims

1. A cosmetic composition comprising:
(a) an alkylresorcinol;
(b) a retinoic acid precursor selected from retinyl esters, retinol, retinal, or mixtures thereof;
(c) a functionalized heteroaromatic compound;
(d) an antioxidant;
(e) 8 to 40% by weight of the cosmetic composition of at least one compatible oil where the oil has a Hansen total solubility parameter from 16.5 to 22 and where the oil is selected from isopropyl myristate, isopropyl palmitate, caprylic/capric triglycerides, or mixtures thereof; and
(f) an emulsifying polymer;
wherein the functionalized heteroaromatic compound comprises a carboxylic acid functionalized heteroaromatic compound, and further wherein the cosmetic composition comprises 0.001 to 5% by weight of the functionalized heteroaromatic compound; wherein the carboxylic acid functionalized heteroaromatic compound comprises nicotinic acid, picolinic acid, nicotinate, niacinamide, picolinamide, or a mixture thereof;
wherein the emulsifying polymer comprises Acrylates/C₁₀₋₃₀ Alkyl Acrylate Crosspolymer, Carbomer, or mixtures thereof,
and further wherein the cosmetic composition comprises 0.01 to 5% by weight of emulsifying polymer.

2. The cosmetic composition according to Claim 1, wherein the alkylresorcinol has an alkyl substituent at the 4-position.

3. The cosmetic composition according to any of the preceding claims, wherein the alkylresorcinol comprises 4-hexylresorcinol, 4-ethylresorcinol, 4-phenylethylresorcinol, 4-cyclopentylresorcinol, 4-cyclohexylresorcinol, 4-octylresorcinol, and mixtures thereof, and further wherein the cosmetic composition comprises 0.001 to 10% by weight of alkylresorcinol.

4. The cosmetic composition according to any of the preceding claims, wherein the cosmetic composition comprises 0.001 to 5% by weight of the retinoic acid precursor.

5. The cosmetic composition according to any of the preceding claims, wherein the antioxidant comprises butylated hydroxytoluene, didodecyl 3,3' thiodipropionate, octadecyl di-t-butyl-4-hydroxyhydrocinnamate, pentaerythrityl tetra-di-t-butyl hydroxyhydrocinnamate, polyphenols, or mixtures thereof and further wherein the cosmetic composition comprises 0.01 to 4%, preferably, 0.02 to 3% by weight of antioxidant.

6. The cosmetic composition according to any of the preceding claims, wherein the cosmetic composition comprises at least 0.2% antioxidant by weight of the cosmetic composition.

7. The cosmetic composition according to any of the preceding claims, wherein the cosmetic composition comprises 10 to 30%, preferably, 12 to 25%, and, more preferably, 15 to 20% by weight compatible oil.

8. The cosmetic composition according to any of the preceding claims, wherein the cosmetic composition is substantially free of additional surfactants.

9. The cosmetic composition according to any of the preceding claims, wherein the cosmetic composition further comprises at least one of 12-hydroxystearic acid, a sunscreen, phospholipid, ceramide, or a mixture thereof.

10. The cosmetic composition according to any of the preceding claims, wherein the cosmetic composition possesses a ΔE value of less than or equal to 6 at 4 weeks at 45°C.

## Patentansprüche

1. Kosmetische Zusammensetzung, umfassend:
(a) ein Alkylresorcin;
(b) einen Retinsäure-Vorläufer, ausgewählt unter Retinylestern, Retinol, Retinal oder Mischungen davon;
(c) eine funktionalisierte heteroaromatische Verbindung;
(d) ein Antioxidans;
(e) 8 bis 40 Gew.-% der kosmetischen Zusammensetzung mindestens eines verträglichen Öls, wobei das Öl einen Hansen-Gesamtlöslichkeitsparameter von 16,5 bis 22 aufweist und wobei das Öl unter Isopropylmyristat, Isopropylpalmitat, Capryl-/ Caprictriglyceriden oder Mischungen davon ausgewählt ist; und
(f) ein emulgierendes Polymer;
wobei die funktionalisierte heteroaromatische Verbindung eine Carbonsäure-funktionalisierte heteroaromatische Verbindung umfasst und wobei die kosmetische Zusammensetzung ferner 0,001 bis 5 Gew.-% der funktionalisierten heteroaromatischen Verbindung umfasst; wobei die Carbonsäure-funktionalisierte heteroaromatische Verbindung Nicotinsäure, Picolinsäure, Nicotinat, Niacinamid, Picolinamid oder eine Mischung davon umfasst;
wobei das emulgierende Polymer Acrylate/C₁₀₋₃₀-Alkylacrylat-Kreuzpolymer, Carbomer oder Mischungen davon umfasst und wobei die kosmetische Zusammensetzung ferner 0,01 bis 5 Gew.-% emulgierendes Polymer umfasst.

2. Kosmetische Zusammensetzung nach Anspruch 1, wobei das Alkylresorcin einen Alkylsubstituenten an der 4-Position aufweist.

3. Kosmetische Zusammensetzung nach einem der vorhergehenden Ansprüche, wobei das Alkylresorcin 4-Hexylresorcin, 4-Ethylresorcin, 4-Phenylethylresorcin, 4-Cyclopentylresorcin, 4-Cyclohexylresorcin, 4-Octylresorcin und Mischungen davon umfasst und wobei die kosmetische Zusammensetzung 0,001 bis 10 Gew.-% Alkylresorcin umfasst.

4. Kosmetische Zusammensetzung nach einem der vorhergehenden Ansprüche, wobei die kosmetische Zusammensetzung 0,001 bis 5 Gew.-% des Retinsäure-Vorläufers enthält.

5. Kosmetische Zusammensetzung nach einem der vorhergehenden Ansprüche, wobei das Antioxidans Butylhydroxytoluol, Didodecyl-3,3'-thiodipropionat, Octadecyl-di-t-butyl-4-hydroxyhydrocinnamat, Pentaerythrityl-tetra-di-t-butylhydroxyhydrocinnamat, Polyphenole oder Mischungen davon umfasst und wobei die kosmetische Zusammensetzung ferner 0,01 bis 4 Gew.-%, vorzugsweise 0,02 bis 3 Gew.-%, Antioxidans umfasst.

6. Kosmetische Zusammensetzung nach einem der vorhergehenden Ansprüche, wobei die kosmetische Zusammensetzung mindestens 0,2% Antioxidans, bezogen auf das Gewicht der kosmetischen Zusammensetzung, umfasst.

7. Kosmetische Zusammensetzung nach einem der vorhergehenden Ansprüche, wobei die kosmetische Zusammensetzung 10 bis 30 Gew.-%, vorzugsweise 12 bis 25 Gew.-% und bevorzugter 15 bis 20 Gew.-% verträgliches Öl umfasst.

8. Kosmetische Zusammensetzung nach einem der vorhergehenden Ansprüche, wobei die kosmetische Zusammensetzung im Wesentlichen frei von zusätzlichen Tensiden ist.

9. Kosmetische Zusammensetzung nach einem der vorhergehenden Ansprüche, wobei die kosmetische Zusammensetzung ferner mindestens eine der folgenden Substanzen umfasst: 12-Hydroxystearinsäure, ein Sonnenschutzmittel, Phospholipid, Ceramid oder eine Mischung davon.

10. Kosmetische Zusammensetzung nach einem der vorhergehenden Ansprüche, wobei die kosmetische Zusammensetzung nach 4 Wochen bei 45°C einen ΔE-Wert von weniger als oder gleich 6 aufweist.

## Revendications

1. Composition cosmétique comprenant :
(a) un alkylrésorcinol ;
(b) un précurseur d'acide rétinoïque choisi parmi les esters de rétinyle, le rétinol, le rétinal, et leurs mélanges ;
(c) un composé hétéroaromatique fonctionnalisé ;
(d) un antioxydant ;
(e) 8 à 40 %, en poids de la composition cosmétique, d'au moins une huile compatible où l'huile a un paramètre de solubilité totale de Hansen de 16,5 à 22 et où l'huile est choisie parmi le myristate d'isopropyle, le palmitate d'isopropyle, les triglycérides capryliques/capriques, et leurs mélanges ; et
(f) un polymère émulsifiant ;
dans laquelle le composé hétéroaromatique fonctionnalisé comprend un composé hétéroaromatique fonctionnalisé par un acide carboxylique, et en outre dans laquelle la composition cosmétique comprend 0,001 à 5 % en poids du composé hétéroaromatique fonctionnalisé ; dans laquelle le composé hétéroaromatique fonctionnalisé par un acide carboxylique comprend l'acide nicotinique, l'acide picolinique, un nicotinate, le niacinamide, le picolinamide, ou un mélange de ceux-ci ;
dans laquelle le polymère émulsifiant comprend un polymère réticulé d'acrylates/acrylate d'alkyle en C₁₀ à C₃₀, un carbomère, ou un mélange de ceux-ci ;
et en outre dans laquelle la composition cosmétique comprend 0,01 à 5 % en poids de polymère émulsifiant.

2. Composition cosmétique selon la revendication 1, dans laquelle l'alkylrésorcinol a un substituant alkyle à la position 4.

3. Composition cosmétique selon l'une quelconque des revendications précédentes, dans laquelle l'alkylrésorcinol comprend le 4-hexylrésorcinol, le 4-éthylrésorcinol, le 4-phényléthylrésorcinol, le 4-cyclopentylrésorcinol, le 4-cyclohexylrésorcinol, le 4-octylrésorcinol, et des mélanges de ceux-ci ; et en outre dans laquelle la composition cosmétique comprend 0,001 à 10 % en poids d'alkylrésorcinol.

4. Composition cosmétique selon l'une quelconque des revendications précédentes, dans laquelle la composition cosmétique comprend 0,001 à 5 % en poids du précurseur d'acide rétinoïque.

5. Composition cosmétique selon l'une quelconque des revendications précédentes, dans laquelle l'antioxydant comprend l'hydroxytoluène butylé, le 3,3'-thiodipropionate de didodécyle, le di-t-butyl-4-hydroxyhydrocinnamate d'octadécyle, le tétra-di-t-butylhydroxyhydrocinnamate de pentaérythrityle, les polyphénols, ou des mélanges de ceux-ci, et en outre dans laquelle la composition cosmétique comprend 0,01 à 4 %, de préférence 0,02 à 3 % en poids d'antioxydant.

6. Composition cosmétique selon l'une quelconque des revendications précédentes, dans laquelle la composition cosmétique comprend au moins 0,2 % d'antioxydant en poids de la composition cosmétique.

7. Composition cosmétique selon l'une quelconque des revendications précédentes, dans laquelle la composition cosmétique comprend 10 à 30 %, de préférence 12 à 25 % et mieux encore 15 à 20 % en poids d'huile compatible.

8. Composition cosmétique selon l'une quelconque des revendications précédentes, dans laquelle la composition cosmétique est pratiquement exempte de tensioactifs additionnels.

9. Composition cosmétique selon l'une quelconque des revendications précédentes, dans laquelle la composition cosmétique comprend en outre au moins l'un parmi l'acide 12-hydroxystéarique, un écran solaire, un phospholipide, un céramide, ou un mélange de ceux-ci.

10. Composition cosmétique selon l'une quelconque des revendications précédentes, dans laquelle la composition cosmétique possède une valeur ΔE inférieure ou égale à 6 après 4 semaines à 45 °C.
